# EUROPÄISCHE PATENTSCHRIFT

(11) **EP 2 985 062 B1**
(45) Veröffentlichungstag und Bekanntmachung des Hinweises auf die Patenterteilung: **17.05.2017**
(21) Anmeldenummer: 15180414.3
(22) Anmeldetag: 10.08.2015
(51) Int. Cl.: B01D 15/22, B01L 3/00, C12N 15/10

(54) **VORRICHTUNG ZUR AUFREINIGUNG VON NUKLEINSÄUREN**
DEVICE FOR PURIFYING NUCLEIC ACIDS
DISPOSITIF DE NETTOYAGE D'ACIDES NUCLEIQUES

(30) Priorität: 13.08.2014 DE 102014216016
(43) Veröffentlichungstag der Anmeldung: 17.02.2016
(73) Patentinhaber: AXAGARIUS GmbH & Co. KG, 52355 Düren (DE)
(72) Erfinder: Greiler, Hubert, 4701 Kettenis (BE); Meusel, Markus, 52146 Würselen (DE)
(74) Vertreter: Davepon, Björn

(56) Entgegenhaltungen:
- EP-A1- 1 136 380
- WO-A1-2006/008085
- WO-A1-2009/157679
- DE-A1- 3 843 610
- DE-U1- 29 803 712
- DE-U1-202004 006 675
- US-A1- 2014 018 529

## Beschreibung

Die Erfindung betriff eine Vorrichtung zur Aufreinigung von Nukleinsäuren aus einem einteilig ausgebildeten Hohlkörper mit einem eine Einlassöffnung umfassenden oberen Abschnitt und einem eine Auslassöffnung umfassenden unteren Abschnitt, wobei in dem Hohlkörper zumindest eine Nukleinsäure-bindende Matrix angeordnet ist. Die Erfindung betrifft ferner ein Verfahren zur Herstellung einer solchen Vorrichtung, ein Verfahren zur Aufreinigung von Nukleinsäuren mithilfe einer erfindungsgemäßen Vorrichtung sowie ein Kit, das eine erfindungsgemäße Vorrichtung enthält.

Die Präparation von Nukleinsäuren gewinnt zunehmend an Bedeutung. So werden RNA und DNA in medizinisch-analytischen, biochemischen und molekularbiologisch arbeitenden Laboratorien benötigt. Die Anwendungsgebiete reichen über die Gentechnologie, Medizin, Veterinärmedizin, Forensik, Molekularbiologie zur Biochemie, sowie von der Grundlagenforschung bis zur angewandten Routinediagnostik.

Für die Isolierung von Nukleinsäuren aus biologischen Proben sind verschiedene Verfahren bekannt. Die ursprünglichen Verfahren schließen das Probenmaterial auf (z.T. mechanisch oder durch enzymatischen Verdau unterstützt) und reinigen die freigesetzten Nukleinsäuren mit Phenol/Chloroform-Gemischen. Die Reinigung kann aber auch technologisch gänzlich andere Verfahren, wie etwa die Präzipitation der Nukleinsäuren oder die Dichtegradientenzentrifugation mit Cäsiumchlorid umfassen.

Alle o.g. Verfahren haben wesentliche Nachteile, wie etwa die Verwendung gesundheitsschädlicher Phenol-/Chloroform-Gemische, oder die aufwändige Nachreinigung der isolierten Nukleinsäuren. Aus diesen Gründen haben sich in den letzten Jahren neue Methoden für die Isolierung von Nukleinsäuren durchgesetzt. Hierzu zählen Silica- und Anionenaustauscher-basierte Techniken. Während die Anionenaustauscher nach wie vor für besonders reine DNA-Präparationen bevorzugt verwendet werden (etwa für Transfektionsexperimente), haben sich die Silica-basierten Techniken als einfache, kostengünstige Methode für eine Vielzahl von Applikationen empfohlen.

Seit den 50er Jahren ist bekannt, dass DNA in Gegenwart chaotroper Salze reversibel an Silikate und andere anorganische Träger bindet. Die Salze bewirken eine Zerstörung der Hydrathülle der Nukleinsäuren und schaffen eine hydrophobe Mikroumgebung. Unter diesen Bedingungen binden die Nukleinsäuren dann an die Silica-Matrix, während Proteine und andere Kontaminanten nicht binden und ausgewaschen werden. Wird hingegen die Bindelösung durch einen Niedrigsalzpuffer oder Wasser ersetzt, findet eine Rehydratisierung der Nukleinsäuren statt, die sich dann bereitwillig von der Silica-Matrix lösen und eluiert werden können. Diese Verfahren werden aufgrund der Abfolge von Nukleinsäurebindung, einem oder mehreren Waschschritten und schließlich der Elution der reinen Nukleinsäuren als "Bind-Wash-Elute" Verfahren bezeichnet.

Üblicherweise werden für die Bind-Wash-Elute Reinigung Zentrifugationssäulen verwendet, in denen als feste Phase eine nukleinsäurebindende Silicamembran oder eine alternative mineralische Bindematrix dienen. Da die einzelnen Prozessierungsschritte, wie etwa das Binden der Nukleinsäuren, üblicherweise in kleinen Labortischzentrifugen stattfinden, werden die entsprechenden Säulen als "Minispin"-Säulen bezeichnet. Diese Säulen sind aus dem Stand der Technik grundsätzlich bekannt und betreffen kleine Säulen, welche beispielsweise in 1,5 mL Eppendorf Reaktionsgefäße eingesetzt und darin in einer Mikrozentrifuge verarbeitet werden können.

Bedingt durch die Bauart der Minispin-Säulen gibt es jedoch eine Reihe von Nachteilen. So fassen diese Säulen üblicherweise nur ca. 1 mL an Volumen, so dass größere Flüssigkeitsmengen nur in einer Reihe aufeinanderfolgender Schritte bearbeitet werden können. Nach dem Stand der Technik wird dieser Nachteil dadurch umgangen, indem auf größere Bindesäulen (üblicherweise als L (large)-Säulen oder XL (xtra large)-Säulen bezeichnet) zurück gegriffen wird. Diese Systeme sind von vielen Herstellern von Nukleinsäure-Reinigungskits wie Sigma, MACHEREY-NAGEL, Qiagen oder Promega im Markt verfügbar und dem Fachmann hinlänglich bekannt. Nachteilig daran ist jedoch, dass durch die größeren Durchmesser der Säulen für die Zentrifugation große Bodenzentrifugen eingesetzt werden müssen. Entgegen den kleinen Tischzentrifugen, die in jedem Labor ohne Einschränkung verwendet werden können, ist dies ein erheblicher Nachteil in Handling, Zeit und auch in der Reinigungseffizienz. So ist die maximal parallel zu bearbeitende Anzahl von Säulen gegenüber den kleinen Minispin-Säulen begrenzt, nachteilig sind weiterhin große Elutionsvolumina (die Nukleinsäuren sind dadurch "verdünnt") und die großen Totvolumina in den Säulen (Verlust an Nukleinsäuren).

Aus dem Stand der Technik sind bislang verschiedene technologische Ansätze bekannt, um diese Nachteile zu umgehen.

In der DE 10 2004 034 474 A1 wird eine Vorrichtung und ein Verfahren zur Nukleinsäurereinigung beschrieben, bei dem der Säulenkörper einer kleinen Zentrifugationssäule, wie einer Minispin-Säule, durch ein aufsteckbares Reservoir vergrößert wird. Die Verbindung wird durch die geometrische Ausgestaltung des Säuleneinlasses und des Reservoirauslasses hergestellt. Die Verbindung erfolgt über Reibung und Pressung, sie ist nicht anderweitig fixiert und leicht lösbar. Bei dieser mehrteiligen Ausführungsform verbleibt das Reservoir bei den ersten Prozessierungsschritten, wie Beladung der Säulen und bei den ersten Waschschritten auf der Zentrifugationssäule. Da die Säulen mit aufgesetztem Reservoir nicht in einer Tischzentrifuge bearbeitet werden können, findet die Bearbeitung der ersten Schritte auf einer Vakuumkammer statt. Dabei werden die Zentrifugationssäulen mit aufgestecktem Reservoir über den Auslass am Boden auf eine Vakuumkammer aufgesteckt. Wird an der Kammer ein Vakuum angelegt, wird die Flüssigkeit aus dem Reservoir über die Silicamembran am Säulenboden in die Vakuumkammer gesaugt. Der Durchlauf wird dabei üblicherweise verworfen. Erst am Ende der Prozessierung wird das Reservoir von der Säule entfernt und die Säule dann weiter in der Zentrifuge, z.B. für den Elutionsschritt, behandelt.

Einen sehr ähnlichen Ansatz beschreibt die US 2010/0222450. Auch hier gibt es ein Reservoir zur Volumenvergrößerung und eine kleine Zentrifugationssäule, wie eine Minispin Säule, die mit dem Reservoir zu einem mehrteiligen Aufbau verbunden wird. Anders aber als in der zuvor genannten DE 10 2004 034 474 A1 wird das Reservoir nicht einfach aufgesteckt, sondern mit Hilfe eines Bajonettverschlusses durch eine Drehbewegung fixiert. Die obere Einlassöffnung der Zentrifugationssäule ist dabei so ausgestaltet, dass über eine Art Kupplung mehrere Formen von Reservoirs angeschlossen werden können. Die Ausgestaltung dieser Kupplung ist bevorzugt als Luer/Lock- oder Luer/Slip-System ausgelegt. Als Reservoir kann beispielsweise auch eine Spritze dienen, andere Ausführungsformen umfassen zylindrische Hohlkörper mit Einlass- und Auslassöffnung, wobei die Verbindung zur Zentrifugationssäule über die auslassseitige Ausgestaltung des Reservoirs erfolgt. Die Kupplung sorgt bei diesen Ausführungsformen für eine Anbindung von Zentrifugationssäule und dem Reservoir.

Die Prozessierung von kleinen Minispin-Säulen für die Extraktion von RNA aus biologischen Materialien ohne die Verwendung eines volumenvergrößernden Reservoirs wird in der US 6,218,531 beschrieben. Hier werden übliche Minispin-Säulen gemäß dem "Bind-Wash-Elute" Verfahren unter Vakuum prozessiert. Geeignete Vakuumkammern sind dem Fachmann bekannt und im Handel verfügbar (siehe z.B. Promega Vac-Man Laboratory Vacuum Manifold, Cat. No A7231, Promega Corporation, Madison, WI, USA). Lediglich die Elution der an die Silicamembran gebundenen RNA erfolgt in einer Tischzentrifuge. Die Verwendung eines Reservoirs oder mehrteiligen Aufbaus ist in der US 6,128,531 nicht offenbart. Nachteilig bei diesem Verfahren ist, dass die Flüssigkeitsmengen, die bearbeitet werden können, durch das eingeschränkte Volumen der Minispin-Säule limitiert sind.

Die EP 1049801 beschreibt ebenso die Nukleinsäureisolierung mittels Plastiksäulen unter Vakuum. Dabei sind in einem Plastikkörper auf einer Polyethylenfritte als mechanischer Unterstützung verschiedene hydrophobe Membranen angeordnet. Die Säule wird über eine Luerverbindung mit einer Vakuumkammer verbunden. Sämtliche Isolierungsschritte finden unter Vakuum statt. Dabei erfolgt das Abnehmen der abgelösten (eluierten) Nukleinsäuren von derselben Seite der Membran, von der sie der Membran zugeführt wurden.

In der DE 20 2004 006 675 U1 wird eine Vorrichtung zur Reinigung von Nukleinsäuren beschrieben, bei der ein erster Hohlkörper mit einem zweiten Hohlkörper reversibel verbunden ist. Die Verbindung ist beispielsweise als Verschraubung ausgeformt, z.B. durch ein außen- oder innenliegendes Schraubgewinde. In weiteren Ausführungsformen werden die beiden Hohlkörper durch Presskräfte miteinander verbunden, z.B. durch eine Steckverbindung. Der zweite Hohlkörper ist dabei eine kleine Zentrifugationssäule mit einem Nukleinsäure-bindenden Material, der erste Hohlkörper übernimmt dabei die Funktion eines Reservoirs, so dass auch bei dieser Anordnung zunächst größere Flüssigkeitsmengen bearbeitet werden können. Nachteilig bei dieser Anordnung ist die Notwendigkeit, die miteinander verbundenen Hohlkörper bedingt durch die Dimension des Reservoirs in einer großen Bodenzentrifuge zu bearbeiten. Das Verfahren ist damit zeit- und arbeitsintensiv.

Ein sehr ähnlicher Ansatz wird in der EP 2055385 beschrieben. Hier wird eine Minispin-Säule mit dem Nukleinsäure-bindenden Material über einen Adapter mit einem Reservoir zu einem mehrteiligen Aufbau verbunden. Auch hier erfolgt die Prozessierung in einer großen Standzentrifuge. Erst für die Elution kann die Minispin-Säule vom Reservoir getrennt und dann in einer Tischzentrifuge bearbeitet werden.

Die Verwendung eines Reservoirs zur Volumenvergrößerung ohne die Notwendigkeit mehrere Bauteile reversibel und zumindest zeitweise miteinander zu verbinden, ist in der DE 29803712 U1 beschrieben. Auch hier wird eine Vorrichtung zur Isolierung von Nukleinsäuren beschrieben. Der einteilige Aufbau ist durch ein großvolumiges Reservoir im oberen Teil der Säule und einen kleinen unteren Teil, der die Silikamembran enthält, gekennzeichnet. Durch den einteiligen Aufbau entfällt die Notwendigkeit mehrere Bauteile gas- und flüssigkeitsdicht miteinander zu verbinden. Das Reservoir erlaubt es, großvolumige oder stark verdünnte Proben direkt zu bearbeiten. Die kleine Silicamembran im unteren Teil der Säule erlaubt kleine Elutionsvolumina und verringert das Totvolumen. Nachteilig ist hier allerdings, dass die Säule durch den groß dimensionierten Reservoirteil nur in großen Standzentrifugen prozessiert werden kann.

Wie dem Stand der Technik zu entnehmen ist, gibt es eine Vielzahl von Vorrichtungen und Verfahren mit mehrteiligen Ausführungsformen. Um den Nachteil großdimensionierter Säulen zu umgehen, werden kleine Minispin-Säulen über verschiedenste Ausgestaltungen mit Reservoirs zur Volumenvergrößerung verbunden. Bei diesen mehrteiligen Ausführungsformen kommen verschiedenste Verbindungstechniken zur Anwendung um die Minispin-Säulen zur Nukleinsäurebindung mit den Reservoirs zu verbinden.
Einteilige Ausführungsformen haben entweder den Nachteil eines kleinen Volumens, so dass eine Vielzahl von Beladungsschritten erforderlich ist, oder nehmen den Nachteil eines großen Säulendurchmessers in Kauf, der die Prozessierung in einfachen Tischzentrifugen ausschließt.
Es ist daher eine Aufgabe der vorliegenden Erfindung, eine Vorrichtung zur Aufreinigung von Nukleinsäuren der eingangs genannten Art zur Verfügung zu stellen, die die Verarbeitung größerer Flüssigkeitsmengen erlaubt und dabei gleichzeitig einfach in der Handhabung ist und insbesondere eine Weiterverarbeitung in einer Tischzentrifunge ermöglicht.

Die Aufgabe wird erfindungsgemäß gelöst durch eine Vorrichtung zur Aufreinigung von Nukleinsäuren aus einem einteilig ausgebildeten Hohlkörper mit einem eine Einlassöffnung umfassenden oberen Abschnitt und einem eine Auslassöffnung umfassenden unteren Abschnitt, wobei in dem Hohlkörper zumindest eine Nukleinsäure-bindende Matrix angeordnet ist, wobei die Vorrichtung dadurch gekennzeichnet ist, dass zwischen dem oberen Abschnitt und dem unteren Abschnitt eine Sollbruchstelle vorgesehen und die Nukleinsäure-bindende Matrix im unteren Abschnitt angeordnet ist, wobei das Volumen des oberen Abschnitts zumindest dem 5-fachen Volumen des unteren Abschnitts entspricht. Mit anderen Worten wird also eine Reservoir-Filtersäule in einer einteiligen Ausgestaltung mit einer Sollbruchstelle zur Verfügung gestellt, bei der der Abschnitt oberhalb der Sollbruchstelle ("Reservoir") zur Aufnahme größerer Probenvolumina dient und nach Prozessierung der großen Probenvolumina und gewünschtenfalls weiterer Waschschritte beispielsweise durch händisches Abbrechen entfernt werden kann. Eingesetzt werden Sollbruchstellen häufig bei medizinisch pharmazeutischen Verpackungen und Behältern. So beschreibt die EP 1136380 beispielsweise einen Einwegbehälter für medizinisch pharmazeutische Anwendungen mit umlaufender Sollbruchstelle. Solche Sollbruchstellen können unter anderem im Rahmen der vorliegenden Erfindung verwendet werden.
Der Abschnitt oberhalb der Sollbruchstelle, der im Weiteren auch als "Reservoir" bezeichnet wird, kann während der Trennung von Reservoir und unterem Abschnitt an der Sollbruchstelle zugleich als Hebel fungieren, sodass zum Abbrechen kein zusätzliches Werkzeug vonnöten ist. Nach dem Abbrechen des oberen Abschnitts, also des Reservoirs, kann der untere Abschnitt, der die mit Nukleinsäuren beladene Nukleinsäure-bindende Matrix umfasst, in einer Tischzentrifunge weiterverarbeitet werden. Das Reservoir kann nach dem Entfernen vom unteren Abschnitt verworfen werden.

Anders als bei zweiteilig ausgebildeten Konstruktionen, die vor der Verwendung noch zusammengesteckt oder verschraubt werden müssen entfällt bei der erfindungsgemäßen Vorrichtung dieser zusätzliche Schritt, wodurch die Handhabung vereinfacht wird.

Zudem ist bei der erfindungsgemäßen Vorrichtung sichergestellt, dass die Verbindung zwischen dem Reservoir und dem unteren Abschnitt gas- und flüssigkeitsdicht ist. Die bekannten mehrteiligen Ausführungen müssen sicherstellen, dass die einzelnen Komponenten und Teile gas- und flüssigkeitsdicht miteinander verbunden werden. Dies ist für die korrekte Funktion unter Vakuum unerlässlich. Der Unterdruck an dem Säulenausgang saugt die Flüssigkeit aus dem Reservoir über die nukleinsäurebindende Matrix wenn der Atmosphärendruck als treibende Kraft über die offene Seite des Reservoirs auf die Flüssigkeit wirkt. Kann der Atmosphärendruck durch Lecks oder undichte Verbindungen unterhalb der Flüssigkeitsoberfläche wirken, wird der Fluss und damit die Filtration/Bindung reduziert oder unterbunden. Zudem kann es hierdurch auch zu ungewollten Kontaminationen kommen. Dieses Problem kann bei den bekannten zweiteiligen Ausgestaltungen beispielsweise dadurch entstehen, dass die Verbindung der beiden Bauteile nicht völlig dicht ist, sei es wegen unzureichend präziser Fertigung der Bauteile oder durch mangelhaften Zusammenbau.

Unter einer "Nukleinsäure-bindenden Matrix" wird im Rahmen der vorliegenden Erfindung ein festes Material verstanden, das geeignet ist, Nukleinsäuren aus einer flüssigen Matrix abzutrennen. Die Abtrennung kann dabei mechanisch erfolgen, wie bei einem Filter, über physikalische und/ oder chemische Wechselwirkungen wie Adsorption. Kombinierte Mechanismen zur Bindung der Nukleinsäuren wie beispielsweise mechanisch und adsorptiv sind also explizit eingeschlossen.

Es versteht sich, dass die Zweckangabe "zur Aufreinigung von Nukleinsäuren" nicht beschränkend zu verstehen ist, sondern dass erfindungsgemäß sämtliche Vorrichtungen geschützt sind, die sich grundsätzlich zu diesem Zweck eignen, selbst wenn sie letztlich zu einem anderen Zweck eingesetzt werden. So kann die Vorrichtung beispielsweise zur Abtrennung von Biomolekülen jedweder Art, als Filter oder auch zur Abtrennung von nicht-Biomolekülen verwendet werden.

Die erfindungsgemäße Vorrichtung umfasst den Hohlkörper in dem die Nukleinsäure-bindende Matrix angeordnet ist, wobei die Vorrichtung durchaus noch weitere Bauteile umfassen kann, wie beispielsweise Dichtungen, Fritten, Vorrichtungen zur Fixierung der Nukleinsäure-bindenden Matrix und dergleichen.

Ein weiterer Gegenstand der vorliegenden Erfindung betrifft ein Verfahren zur Herstellung einer erfindungsgemäßen Vorrichtung zur Aufreinigung von Nukleinsäuren, wobei ein einteiliger Hohlkörper mit einer Einlassöffnung und einer Auslassöffnung und einer Sollbruchstelle erzeugt und anschließend in den Hohlkörper zwischen der Sollbruchstelle und der Auslassöffnung zumindest eine Nukleinsäure-bindende Matrix angeordnet wird.

Die vorliegende Erfindung betrifft ferner ein Verfahren zur Aufreinigung von Nukleinsäuren aus einer flüssigen Nukleinsäure-haltigen Probe umfassend die folgenden Schritte:
a) Bereitstellen der flüssigen Nukleinsäuren enthaltenden Probe und Einstellung der Bindebedingungen, damit die Nukleinsäuren an die Nukleinsäure-bindende Matrix binden können;
b) Überführen der Probe in eine erfindungsgemäße Vorrichtung durch die Einlassöffnung der Vorrichtung;
c) Hindurchführen der Probe durch die Nukleinsäure-bindende Matrix, wobei die Nukleinsäuren an die Nukleinsäure-bindende Matrix binden und wobei das Hindurchführen insbesondere unter Anlegen von Vakuum an der Auslassöffnung der Vorrichtung erfolgt;
d) optionales Waschen der Nukleinsäure-bindenden Matrix;
e) Trennen des oberen Abschnitts vom unteren Abschnitt entlang der Sollbruchstelle, insbesondere durch händisches Abbrechen;
f) optionales Waschen der Nukleinsäure-bindenden Matrix;
g) Elution der Nukleinsäuren von der Nukleinsäure-bindenden Matrix und Auffangen der eluierten Nukleinsäuren in einem separaten Auffanggefäß, wobei die Elution insbesondere in einer Zentrifuge durchgeführt wird.

Die Erfindung ist zudem gerichtet auf ein Kit zur Aufreinigung von Nukleinsäuren aus einer Nukleinsäure-haltigen flüssigen Probe umfassend eine erfindungsgemäße Vorrichtung, sowie eine Bedienungsanleitung zur Durchführung des erfindungsgemäßen Verfahrens, und/ oder zur Aufreinigung von Nukleinsäuren geeignete Mittel, wie mindestens einen Lyse- und/oder Bindepuffer, Waschpuffer und/oder Elutionspuffer.

Nach einer bevorzugten Weiterbildung der erfindungsgemäßen Vorrichtung entspricht das Volumen des oberen Abschnitts zumindest dem 20-fachen des Volumens des unteren Abschnitts, insbesondere wenigstens dem 40-fachen, besonders bevorzugt wenigstens dem 50-fachen. Ausführungen mit einem 55-fachen Volumen und größer sind ebenfalls möglich. Unter Volumen wird im Rahmen der vorliegenden Erfindung das Hohlvolumen verstanden, dass der jeweilige Abschnitt der Vorrichtung aufweist. Bei einem zylindrisch ausgeformten oberen Abschnitt ist dessen Volumen beispielsweise das von dem Zylinder eingefasste Volumen begrenzt durch den Rand der Einlassöffnung und auf der gegenüberliegenden Seite begrenzt durch die Sollbruchstelle.

Das unterschiedliche Volumen von oberem und unterem Abschnitt kann beispielsweise durch Wahl unterschiedlicher Durchmesser und/ oder unterschiedlicher Längenausdehnung von oberem und unterem Abschnitt realisiert werden.

Absolut betrachtet kann das Volumen des unteren Abschnitts im wesentlichen dem Volumen handelsüblicher Mini-Spinsäulen entsprechen, also beispielsweise etwa 1 mL, insbesondere 0,5 bis 1,5 mL. Das Volumen des oberen Abschnitts, also des Reservoirs, kann unabhängig hiervon beispielsweise 5 bis 100 mL betragen, insbesondere 10 bis 80 mL, vorzugsweise 20 bis 40 mL, wobei auch andere Volumina möglich sind, wenn dies für bestimmte Anwendungszwecke erforderlich ist. Die Größe insbesondere des Reservoirs kann in weiten Bereichen variieren, ohne dass die Weiterverarbeitungsschritte bei der Verwendung der erfindungsgemäßen Vorrichtung nennenswert angepasst werden müssen, da vor dem Zentrifugieren das Reservoir an der Sollbruchstelle abgetrennt wird.

Bei der erfindungsgemäßen Vorrichtung können der obere Abschnitt und der untere Abschnitt unabhängig voneinander einen runden oder rechteckigen, wie beispielsweise einen quadratischen Querschnitt besitzen. Zur Vereinfachung der Herstellung ist die Wahl einer runden Querschnittsform bevorzugt.

Der obere Abschnitt und der untere Abschnitt können unabhängig voneinander beispielsweise eine zylindrische oder konische Form besitzen. Insbesondere der obere Abschnitt kann eine sich in Richtung der Einlassöffnung aufweitende konische Form besitzen, wodurch das Einfüllen größerer Probenvolumina erleichtert wird.

Nach einer weiter bevorzugten Ausgestaltung der erfindungsgemäßen Vorrichtung ist der obere Abschnitt im Wesentlichen zylindrisch ausgestaltet, wobei sich der Querschnitt oberhalb der Sollbruchstelle in Richtung auf den unteren Abschnitt verjüngt, vorzugsweise derart, dass sich der Außendurchmesser des oberen Abschnitts bis etwa auf den Außendurchmesser des unteren Abschnitts unmittelbar unterhalb der Sollbruchstelle verjüngt, vorzugsweise in Form einer konischen Verjüngung. Dies ist von Vorteil, weil hierdurch sichergestellt werden kann, dass die im Reservoir eingefüllte flüssige Probe praktisch rückstandsfrei in den unteren Abschnitt fließt.

Es ist weiterhin vorteilhaft, dass der untere Abschnitt unmittelbar unterhalb der Sollbruchstelle mit einer wenigstens eine Stufe aufweisenden Verjüngung ausgerüstet ist, insbesondere mit einer zwei Stufen aufweisenden Verjüngung. Durch die stufenförmige Verjüngung werden an der Außenseite des unteren Abschnitts Rastflächen gebildet, mit denen der untere Abschnitt nach dem Entfernen des oberen Abschnitts in die Aufnahme einer Zentrifuge eingesetzt werden kann, insbesondere einer Tischzentrifuge. Dabei ist die Innenwandung des unteren Abschnitts im Bereich der außenseitigen stufenförmigen Verjüngung vorzugsweise nicht stufenförmig sondern mit einer schräg verlaufenden Verjüngung versehen. Dadurch wird sichergestellt, dass möglichst keine Reste der flüssigen Probe anhaften können.

Nach einer vorteilhaften Weiterbildung der erfindungsgemäßen Vorrichtung ist der obere Abschnitt an der Eingangsöffnung mit einer umlaufenden Randwulst versehen. Hierdurch kann der Vorrichtung eine höhere Stabilität verliehen werden. Zudem kann die Randwulst an Anschlagfläche dienen, um die Vorrichtung in eine Lochhalterung einzusetzen, die einen Lochdurchmesser besitzt, der im Wesentlichen demjenigen des oberen Abschnitts entspricht, jedoch kleiner ist als der Außendurchmesser der Randwulst.

Der Hohlkörper kann aus allen prinzipiell geeigneten Materialien aufgebaut sein. Geeignete Materialien sollten eine gewisse mechanische Stabilität aufweisen, im Hinblick auf die typischerweise verwendeten Chemikalien weitestgehend inert sein und nur eine geringe Nukleinsäurebindung aufweisen. Ihre mechanischen Eigenschaften sollten zudem eine leichte Trennung von Ober- und Unterteil an der Sollbruchstelle erlauben. Bevorzugt ist der Hohlkörper aus einem Kunststoff aufgebaut. Geeignete Kunststoffe können aus Thermoplasten, Duroplasten und Elastomeren ausgewählt werden. Der Kunststoff ist insbesondere ausgewählt aus Polyolefinen wie Polyethylen oder Polypropylen, aus biobasierten Kunststoffen wie Polyhydroxybuttersäure oder Polylactaten, Polyamiden wie Nylon, Polyimiden, Acetalen, Polyvinylchlorid, Polytetrafluorethylen, Polyestern, Polycarbonaten, Polymethyl(meth)acrylaten, Acrylnitril-Butatien-Styrol Terpolymerisat (ABS), Polystyrol oder beliebigen Mischungen und/ oder Copolymeren von diesen.

Weiterhin kann der Hohlkörper insbesondere auf seiner inneren Oberfläche zumindest abschnittsweise oder auch vollständig mit einer Beschichtung versehen sein, die beispielsweise die Oberflächenspannung in Bezug auf Wasser reduziert oder erhöht. Solche Beschichtungen können in an sich bekannter Weise beispielsweise durch eine Silanisierung der Oberfläche oder andere dem Fachmann bekannte Maßnahmen erfolgen.

Der Hohlkörper kann beispielsweise über ein Spritzgussverfahren, durch Blasformen, Gießtechnik mit Silikonformen oder Methoden des Rapid-Prototyping (z.B. 3D-Druck, Fused Deposite Modeling, Lasersintern oder Elektronenstrahlschmelzen) hergestellt sein. Ein Spritzgussverfahren ist insofern vorteilhaft, weil sich hierdurch der Hohlkörper mit ausreichender Präzision, hoher Reproduktionsgenauigkeit und in hohen Stückzahlen herstellen lässt. Zudem kann die Sollbruchstelle vergleichsweise einfach schon im Spritzgussprozess integriert werden. Selbstverständlich kann die Sollbruchstelle auch nach der Herstellung des Hohlkörpers angebracht werden, beispielsweise durch spanabhebende Maßnahmen oder mithilfe eines Lasers. Alternativ kann der erfindungsgemäße Hohlkörper auch durch zerspanende, formgebende Bearbeitungsverfahren hergestellt werden. Diese umfassen beispielsweise Drehen, Fräsen, Bohren, Sägen und Schleifen.

Als Nukleinsäure-bindende Matrix kann im Rahmen der vorliegenden Erfindung jedwedes Material eingesetzt werden, das dem Fachmann zu dem genannten Zweck bekannt ist. Beispiele für mineralische hierfür geeignete Materialien sind poröse oder nicht poröse Substanzen wie Silika, Glas, Quarz, Zeolithe oder Metalloxide. Die Materialien können in Form von Membranen, Fasern, Geweben, Sintern, Sieben oder auch partikulär vorliegen. Ebenso geeignet sind Anionenaustauscherharze oder Materialien, die mit Anionenaustauscherfunktionen chemisch modifiziert sind. Diese können als poröse Membran, in partikulärer Form oder in anderer, an sich bekannter Weise verwendet werden. Bei den mineralischen Trägern bieten sich insbesondere Glasfaser- Quartzfasermembranen an, da diese kostengünstig in der Herstellung sind und gute Bindungseigenschaften zu Nukleinsäuren besitzen. Glasfaserfilter/-membranen sind typischerweise aus Mikro-Glasfasern aufgebaut. Im technischen Bereich werden solche Filter üblicherweise als Wasser-oder Luftfilter eingesetzt. Die Bindematrix kann aber auch partikuläres Material (Silicapartikel, Silicagel) umfassen. Partikuläres Material wird bevorzugt zwischen zwei flüssigkeitspermeablen Schichten (z.B. Kunststofffritten oder Filter) fixiert, die den Durchtritt der Partikel verhindern.

Werden Membranen oder Filter eingesetzt, kann die Porengröße der Membran auf den jeweiligen Anwendungszweck hin angepasst werden. Bedingt durch den Aufbau aus einem Fasernetzwerk kann man für Glasfasermembranen oder-fliese i.d.R. keine definierte Porengröße angegeben werden, auch wenn in der Literatur üblicherweise Werte von z.B. 2-5 µm angegeben werden. Die Filterleistung solcher Materialien wird daher üblicherweise über das Rückhaltevermögen gemäß DIN EN 1822-3 (Januar 2011) definiert. Übliche Rückhaltewerte (d.h. Partikelgrößen die zurückgehalten werden) liegen beispielsweise im Bereich von 0,1 bis 5 µm, bevorzugt von 0,5 bis 4 µm, weiter bevorzugt 1 bis 3 µm.

Nach einer bevorzugten Ausführungsform der erfindungsgemäßen Vorrichtung kann die Nukleinsäure-bindende Matrix auf einem Träger fixiert sein. Hierfür eignet sich insbesondere eine Trägerfritte, beispielsweise aus Glas, Keramik oder Kunststoff, wobei als Werkstoffe grundsätzlich dieselben Materialien eingesetzt werden können, aus denen der Hohlkörper aufgebaut ist, wobei die Materialwahl jedoch unabhängig von der des Hohlkörper erfolgen kann. Eine bevorzugte Ausführungsform sieht vor, die Trägerfritte aus gesintertem Kunststoff zu fertigen. Die Fixierung der Nukleinsäure-bindenden Matrix auf dem Träger kann beispielsweise über einen Spannring erfolgen, der durch Klemmung, Klebung oder auf anderen Wegen im Hohlkörper fixiert wird. Durch diesen Aufbau ist es möglich, unterschiedliche Nukleinsäure-bindende Matrizes für denselben Hohlkörper herzustellen und/ oder anzubieten. Der Zusammenbau kann dann bedarfsgerecht erfolgen und sogar vom eigentlichen Verbraucher vorgenommen werden.

Nach einer besonders bevorzugten Ausführungsform der erfindungsgemäßen Vorrichtung ist die Sollbruchstelle durch eine im Wesentlichen durchgehend den Hohlkörper umlaufende Schwächungslinie ausgebildet, insbesondere als die Wandungsstärke des Hohlkörpers reduzierende Nut. Besonders bevorzugt besitzt die Nut die Form einer Keilnut, da sich hierdurch der obere und untere Abschnitt besonders einfach, das heißt ohne großen Kraftaufwand, voneinander trennen lassen.

Ganz besonders bevorzugt ist es, dass die Sollbruchstelle derart ausgestaltet ist, dass sie eine Trennung des oberen vom unteren Abschnitt ohne Zuhilfenahme von Werkzeugen ermöglicht.

Im Rahmen des erfindungsgemäßen Verfahrens können insbesondere die Schritte, die ein großes Flüssigkeitsvolumen erfordern, unter Vakuum auf handelsüblichen Vakuumkammern durchgeführt werden, beispielsweise durch Anlegen eines Unterdrucks von 500 mbar oder darunter, insbesondere 300 mbar oder darunter. Dazu kann die Auslassöffnung der Vorrichtung so ausgestaltet sein, dass er auf die handelsüblichen Vakuumkammern mit handelsüblichen Adaptern passt. Bevorzugt kommen dem Fachmann an sich bekannte Luer/Lock oder Luer/Slip Adapter zum Einsatz. Insofern ist in einer bevorzugten Ausführungsform der erfindungsgemäßen Vorrichtung die Auslassöffnung derart ausgestaltet, dass sie sich zum Aufstecken auf eine Vakuumkammer eignet, wobei die Auslassöffnung vorzugsweise als Luer-Male Adapter ausgestaltet ist. Dies ist besonders vorteilhaft, weil durch diese Formgebung die Notwendigkeit des Einsatzes von Adaptern oder dergleichen entfällt und der untere Abschnitt direkt auf den Anschluss einer Vakuumkammer aufgesteckt werden kann.

Als Probenmaterial eignet sich jedwedes biologische Material, das Nukleinsäuren enthält, wie tierisches oder menschliches Gewebe, Gewebeteile, Körperflüssigkeiten wie Speichel, Sputum, Liquor, Vollblut, Serum oder Plasma. Bakterien, Hefen und andere Pilze oder Viren werden ebenso unter "Probenmaterial" verstanden, wie auch PCR-Amplifikationsreaktionen, die Primer und DNA Fragmente enthalten, oder Zellkulturüberstände. Probenmaterial kann auch Umwelt- oder Lebensmittelproben umfassen. Auch künstliches Probenmaterial, z.B. mit synthetisch oder in-vitro erzeugten Nukleinsäuren fällt unter den Anwendungsbereich der vorliegenden Erfindung.

Nukleinsäuren im Sinne der Erfindung umfassen jede Form von Nukleinsäuren: RNA, DNA, bakteriellen, viralen, tierischen oder pflanzlichen Ursprungs. Weiterhin werden unter Nukleinsäuren lang- und kurzkettige, einzel- und doppelstränge, lineare und verzweigte natürliche Nukleinsäuren (z.B. genomische DNA, mRNA, miRNA, siRNA), ebenso wie künstliche Nukleinsäuren, etwa PCR-Fragmente verstanden, genauso wie freie oder zellgebundene oder in-vitro erzeugte (z.B. cDNA) Nukleinsäuren.

Das erfindungsgemäße Verfahren ist nicht auf ein bestimmtes Prinzip der Nukleinsäurereinigung beschränkt. Dem Stand der Technik sind verschiedenartige Verfahren zu entnehmen, die angewendet werden können und dem Fachmann bekannt sind. Diese umfassen beispielsweise die Verwendung von Anionenaustauschern, die Verwendung chaotroper Salze, die Verwendung antichaotroper Salze, Ausfällungen (z.B. Fällungen mit Polyethylenglycol), Filtration, die Ausnutzung hydrophober Wechselwirkungen für die Nukleinsäurebindung und andere Verfahren.

Eine besonders bevorzugte Ausführungsform der erfindungsgemäßen Vorrichtung beziehungsweise des Verfahrens sieht die Verwendung chaotroper Salze in der Kombination mit einer Silicamembran als Nukleinsäure-bindende Matrix vor. Gemäß geltender Theorien zerstören die chaotropen Salze die geordnete Wasserstruktur um die Nukleinsäuren, so dass diese an Oberflächen mineralischer Träger, insb. an Glas- und Silicaträger (Siliziumdioxid in Form von Faser oder Partikeln, Glasvlies, Silicagel, Zeolith, etc.) binden können. Chaotrope Salze sind so definiert, dass sie Proteine denaturieren, die Löslichkeit unpolarer Substanzen in Wasser erhöhen sowie hydrophobe Wechselwirkungen zerstören. Die Stärke des chaotropen Charakters eines Salzes ist in der s.g. Hofmeister-Reihe beschrieben. Im Rahmen der vorliegenden Erfindung können beispielsweise Natriumperchlorat, Natriumiodid, Guanidinium isothiocyanat und Guanidinium hydrochlorid oder auch Kombination hiervon als chaotrope Salze verwendet werden.

Im Rahmen des erfindungsgemäßen Verfahrens kann die biologische Probe zunächst aufgeschlossen, d.h. lysiert, werden um die Nukleinsäuren aus dem Material freizusetzen. Der Aufschluss kann einen mechanischen, chemischen und/ oder enzymatischen Aufschluss umfassen. Der Aufschluss der Probe wird häufig durch eine geeignete Pufferchemie unterstützt, die beispielsweise Detergenzien beinhaltet. Geeignete Lysebedingungen sind dem Fachmann bekannt.

Wird das aufgeschlossene (lysierte) Material mit den freigesetzten Nukleinsäuren in der erfindungsgemäße Vorrichtung gegeben, so erfolgt der Probendurchtritt durch die Nukleinsäurebindende Matrix. Dies kann beispielsweise durch Schwerkraft, Zentrifugation, Unter- und Überdruck erfolgen. Bevorzugt wird die Vorrichtung bei diesem Verfahrensschritt auf eine Vakuumkammer aufgesteckt, die, bei Anlegen eines geeigneten Unterdrucks, die Flüssigkeit aus dem Reservoir über die Nukleinsäure-bindende Matrix in die Vakuumkammer saugt. Während dieses Schrittes binden die Nukleinsäuren an die Nukleinsäure-bindende Matrix. Die Bindung in Gegenwart der oben beschriebenen chaotropen Salze (üblich sind beispielsweise Salzkonzentrationen von 1 bis 6 mol/L), ggf. unterstützt durch weitere Bestandteile der Bindelösung (wie etwa kurzkettige Alkohole, wie Methanol, Ethanol, Propanol und dergleichen), ist dem Fachmann bekannt. Andere Bindeprinzipien, wie etwa die Bindung an Anionenaustauscher, sind ebenfalls möglich.

Nach diesem Schritt befinden sich die Nukleinsäuren an der Nukleinsäure-bindende Matrix im unteren Teil der erfindungsgemäßen Vorrichtung, während die Probenflüssigkeit abgetrennt wurde. Der untere Abschnitt mit den Nukleinsäuren kann nun vom oberen Abschnitt der Vorrichtung getrennt und weiter aufbereitet werden. Die Abtrennung erfolgt durch einfaches Abbrechen an der dafür vorgesehenen Sollbruchstelle. Der untere Teil der Säule mit den gebundenen Nukleinsäuren wird weiter verarbeitet, während das Reservoir verworfen wird. Praktischerweise erfolgt die Trennung nach den Waschschritten oder vor der Elution.

Da den Nukleinsäuren üblicherweise noch Verunreinigungen anhaften, die beim Bindeschritt auch an die feste Phase gebunden werden, kann sich an dieser Stelle zweckmäßigerweise ein Waschschritt anschließen. Solche Waschschritte und geeignete Lösungen sind dem Fachmann bekannt. Die Waschbedingungen werden typischerweise so eingestellt, dass die Bindung der Nukleinsäuren an die feste Phase, also an die Nukleinsäure-bindende Matrix, nicht gelöst wird. Verunreinigungen werden jedoch entfernt. Im Beispiel der Verwendung chaotroper Salze schließt sich an die Bindung üblicherweise ein Waschschritt mit hoher (Chaotrop-)Salz Konzentration an, in welchem Detergenzien und Proteine entfernt werden, sowie ein weiterer Waschschritt ohne/mit wenig Salz und hoher Alkoholkonzentration. Mit diesem Schritt werden Reste der chaotropen Salze entfernt, die Bindung der Nukleinsäuren an die feste Phase bleibt in Gegenwart hoher Alkoholkonzentrationen hingegen erhalten. Unter einer hohen (Chaotrop-)Salz Konzentration wird in diesem Zusammenhang beispielsweise eine wässrige Lösung von wenigstens 0,2 mol/L Salz verstanden, bevorzugt von wenigstens 0,5 mol/L bis 5 mol/L. In dem weiteren Waschschritt wird unter "wenig Salz" eine Salzkonzentration von höchstens 1 mol/L verstanden, vorzugsweise von höchstens 0,1 mol/L und unter "hoher Alkoholkonzentration" wird eine Alkoholkonzentration von wenigstens 50 Gew.-%, verstanden, bevorzugt von 60 Gew.-% bis 90 Gew.-%.

Diese Waschschritte können je nach Grad der Verunreinigungen vor der Trennung des unteren Teils der Vorrichtung vom Reservoir erfolgen. Vorteilhaft ist dabei, dass große Volumina der Waschlösungen in einem Schritt aufgetragen und durch die Nukleinsäure-bindende Matrix geleitet werden können. Spätestens vor dem Elutionsschritt der Nukleinsäuren kann das Reservoir der erfindungsgemäßen Vorrichtung vom unteren Abschnitt mit den gebundenen Nukleinsäuren abgetrennt werden.

Nach dem Abtrennen des Reservoirs findet bevorzugt ein weiterer Waschschritt in einer Zentrifuge statt. Durch das Fehlen des Reservoirs kann der die eigentliche Säule darstellende untere Abschnitt der Vorrichtung in üblichen Tischzentrifugen prozessiert werden. Durch die hohen Beschleunigungen, wie sie in üblichen Tischzentrifugen erreicht werden, können Reste der Waschlösungen (z.B. Alkohole) leicht und vollständig entfernt werden wie es unter Vakuum nicht oder nicht so vollständig und effektiv durchzuführen wäre.

In einer weiter bevorzugten Ausgestaltung des erfindungsgemäßen Verfahrens ist vorgesehen, die Säulen vor der Elution nicht noch einmal mit Waschlösung zu beaufschlagen, sondern die Säule nur kurz zu zentrifugieren (beispielsweise für 2 min bei 11.000 x g), um Reste der Waschlösungen zu entfernen. Wird für die Bindung der Nukleinsäuren auf die bekannte Chaotrop-Chemie zurückgegriffen, so erfolgt die Elution üblicherweise in Puffern geringer Ionenstärke (z.B. 5 mM Tris Puffer) oder Wasser. Das Wasser rehydriert die Nukleinsäuren, so dass sich diese von der festen Phase ablösen und im Zuge der Zentrifugation in einem separaten Auffanggefäß aufgefangen werden können.

Wird ein anderes Verfahren zur Bindung der Nukleinsäuren eingesetzt, wie etwa ein Anionenaustauscher, so erfolgt das Waschen und die Elution der Nukleinsäuren unter anderen Bedingungen. Diese Verfahren sind dem Stand der Technik nach für den Fachmann bekannt. Anionenaustauscher werden überwiegend für die Plasmidreinigung im s.g. Midi- oder Maxi Maßstab eingesetzt. Bakterielle Zellen werden dabei durch Zentrifugation geerntet, mit Resuspensionspuffer aufgenommen und unter alkalischen Bedingungen lysiert. Unter diesen Bedingungen denaturieren sowohl chromosomale als auch Plasmid-DNA. Die Zugabe von Kaliumacetat führt zur Neutralisation und zur Bildung eines Präzipitats aus chromosomaler DNA und Zellbruchstücken und Protein. Nur die Plasmid-DNA kann renaturieren und bleibt in Lösung. Nach Abtrennung der unlöslichen Bestandteile wird das bakterielle Lysat auf den Anionenaustauscher gegeben. Bei hoher Salzkonzentration und niedrigem pH Wert bindet das negativ geladene DNA-Rückgrat an die positiv geladenen Anionenaustauschergruppen. Waschschritte mit steigender Salzkonzentration entfernen Kontaminanten. Letztlich wird die gebundene DNA durch ein Anheben des pH Wertes vom Anionenaustauscher eluiert. Sie wird anschließend durch Fällung von den Salzresten befreit.

Die vorliegende Erfindung wird im Folgenden anhand von zwei Zeichnungen und Ausführungsbeispielen näher erörtert. Darin zeigt
- Fig. 1: Eine Ausführungsform einer erfindungsgemäßen Vorrichtung,
- Fig. 2: eine ausschnittsweise Vergrößerung eines Bereichs B der Vorrichtung gemäß Fig. 1, sowie
- Fig. 3: eine konkrete Ausgestaltung der Vorrichtung gemäß Fig. 1 mit Angaben zur Bemaßung.

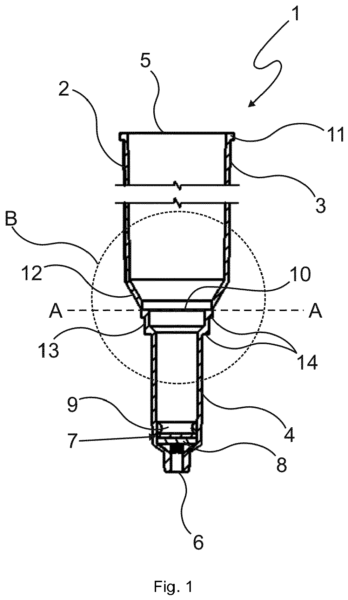

In Fig. 1 ist eine Vorrichtung 1 zur Nukleinsäureisolierung in seitlicher Schnittdarstellung abgebildet. Die Vorrichtung umfasst einen einteilig ausgebildeten Hohlkörper 2 mit rundem Querschnitt und einem oberen Abschnitt 3 sowie einem unteren Abschnitt 4. Der Hohlkörper 2 ist aus Polypropylen als Spritzgussteil gefertigt. Am oberen Abschnitt 3 ist eine Einlassöffnung 5 zum Einfüllen einer flüssigen, Nukleinsäuren enthaltenden Probe vorgesehen. Im unteren Abschnitt 4 ist eine Nukleinsäure-bindende Matrix in Form 7 einer Silikamembran (Glasfaserfilter) mit einem Rückhaltevermögen von 1,4 µm gemäß DIN EN 1822-3 (Januar 2011) auf einer Trägerfritte 8 mithilfe eines Spannrings 9 fixiert. In Durchflussrichtung der Vorrichtung 1 ist unterhalb der Silikamembran 7 eine Auslassöffnung 6 vorgesehen, der als Luer-Male Adapter ausgeformt ist.

Zwischen dem oberen Abschnitt 3 und dem unteren Abschnitt 4 ist eine Sollbruchstelle 10 in Form einer umlaufenden Keilnut ausgebildet, die die händische Trennung des oberen Abschnitts 3 und des unteren Abschnitts 4 entlang der gestrichelten Linie A-A ermöglicht. Der obere Abschnitt 3 weist ein Hohlvolumen von 38,5 mL auf, der untere Abschnitt 4 ein Hohlvolumen von ca. 1 mL. Hiervon sind typischerweise 0,7 mL nutzbar, um ein Überlaufen von Flüssigkeiten über den Rand zu verhindern.

Der obere Abschnitt 3, der auch als Reservoir bezeichnet wird, besitzt an der Einlassöffnung 5 eine umlaufende Randwulst 11. An seinem gegenüberliegenden Ende, also an seiner der Sollbruchstelle 10 zugewandten Seite, ist der obere Abschnitt 3 mit einer konischen Verjüngung 12. Über die konische Verjüngung 12 reduziert sich der Außendurchmesser des oberen Abschnitts 3 bis zur Sollbruchstelle 10 in etwa auf den Außendurchmesser des unteren Abschnitts 4.

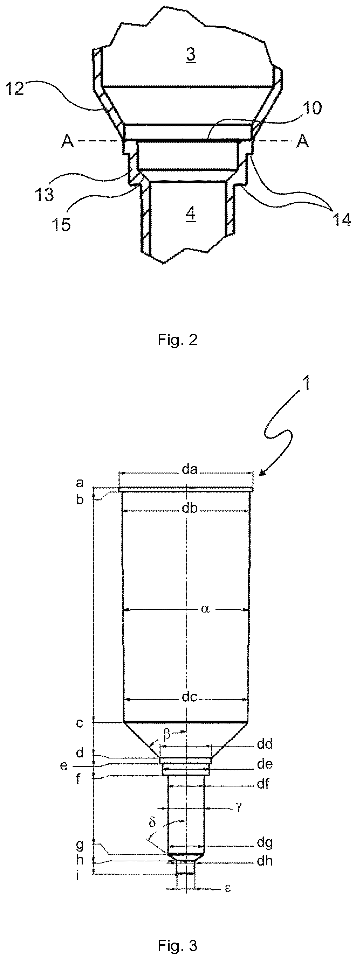

Wie im Kreisausschnitt B der Fig. 1 in Fig. 2 vergrößert dargestellt ist, ist der untere Abschnitt 4 unmittelbar unterhalb der Sollbruchstelle 10 mit einer zwei Stufen aufweisenden Verjüngung 13 ausgerüstet, wodurch an der Außenseite des unteren Abschnitts 4 Rastflächen 14 gebildet sind, mit denen der untere Abschnitt 4 nach dem Entfernen des oberen Abschnitts 3 in die Aufnahme einer Zentrifuge eingesetzt werden kann. Dabei ist die Innenwandung des unteren Abschnitts 4 im Bereich der außenseitigen stufenförmigen Verjüngung 13 vorzugsweise nicht stufenförmig sondern mit einer schräg verlaufenden Verjüngung 15 versehen.

In Fig. 3 ist eine konkrete Ausgestaltung der Vorrichtung 1 gemäß Fig. 1 mit Angaben zur Bemaßung dargestellt. Die jeweiligen Größen sind in der folgenden Tabelle zusammengefasst:

| Position | Länge a bis Position x [mm] | Durchmesser | dx [mm] | Winkel | [°] |
|---|---|---|---|---|---|
| a | - | da | 32 | α | 1° |
| b | 1 | db | 30,6 | β | 45° |
| c | 56,4 | dc | 29,6 | γ | 1,1° |
| d | 65 | dd | 12,4 | δ | 55° |
| e | 66,4 | de | 11,1 | ε | 1,7° |
| f | 69,3 | df | 8,8 | | |
| g | 88,4 | dg | 8,4 | | |
| h | 89,8 | dh | 4,3 | | |
| i | 93,2 | | | | |

Die Längenangaben in der Tabelle geben die Entfernung der jeweiligen Position zur Oberkante der Vorrichtung 1 wider, der Position a. Die Ausdehnung der Abschnitte ergibt sich rechnerisch aus dem Abstand des jeweilgen Abschnittsendes zum Ende des vorhergehenden Abschnitts. Beispielsweise erstreckt sich der Abschnitt a-b vom Beginn der Randwulst 11 - Position a - zum Ende der Randwulst 11 - Position b. Die Durchmesserangaben sind auf die in Fig. 3 dargestellte Position bezogen. Die Winkelangaben beziehen sich auf den Winkel zwischen der Wandung an der bezeichneten Position und der die Vorrichtung 1 durchlaufenden gedachten geometrischen Mittellinie in Durchflussrichtung. Die Hohlvolumina des oberen und unteren Abschnitts 3, 4 entsprechen denjenigen, wie sie zur Fig. 1 angegeben sind.

Es folgen nun mehrere Ausführungsbeispiele zur Trennung von Nukleinsäuren mithilfe der erfindungsgemäßen Vorrichtung 1 und des erfindungsgemäßen Verfahrens.

### BEISPIEL 1:

Protokoll zur Reinigung von Plasmid DNA aus *E. Coli*
1. 200 mL einer *E. coli* XL1 Blue Kultur mit low-copy Vektor und einer OD von 1,8 wurde pelletiert und die Zellen in 7 mL A1 mit RNase A resuspendiert (A1: NucleoSpin Plasmid, REF 740855, MACHEREY-NAGEL, Düren, Deutschland - handelsüblicher Tris/EDTA Resuspensionspuffer mit RNase A).
2. Nach alkalischer Lyse mit 7 mL A2 und Neutralisation mit 8,4 mL A3 wurde das Präzipitat durch Zentrifugation entfernt (10 min, 10,000 x g; NucleoSpin Plasmid, REF 740588, MACHEREY-NAGEL, Düren, Deutschland - A2 handelsüblicher Puffer für die alkalische Lyse von Bakterien mit NaOH/SDS; A3 handelsüblicher Neutralisations- und Bindepuffer mit Kaliumacetat und Guanidinium hydrochlorid).
3. Das klare Lysat wurde über Vakuum auf die erfindungsgemäße Säule geladen und mit 5 mL Waschpuffer AW, sowie 2x 5 mL Waschpuffer A4 unter Vakuum gewaschen (NucleoSpin Plasmid, REF 740855, MACHEREY-NAGEL, Düren, Deutschland - AW handelsüblicher Hochsalz-Waschpuffer mit Guanidinium hydrochlorid und Ethanol; A4 handelsüblicher Alkohol-Waschpuffer mit 80 % Ethanol). Die erfindungsgemäße Säule wurde gemäß Figur 1 mit einer umlaufenden Sollbruchstelle ausgestaltet. Im unteren Teil der Säule, wurden 6 Lagen Silicamembran eingebracht und mit einem Kunststoffspannring in der Säule fixiert.
4. Dann wurde der Reservoirteil entfernt (abgebrochen), der untere Teil der Säule in ein Collection Tube (2 mL) überführt.
5. Die Säule wurde in einer Tischzentrifuge für 2 min bei 11.000 x g getrocknet.
6. Die Elution der DNA erfolgte in 50 µL Elutionspuffer AE (5 mM Tris/HCl) durch Zentrifugation für 1 min bei 11.000 x g.

### Ergebnis:

Die photometrische Vermessung des Eluates ergab eine Ausbeute von 11,8 µg und Reinheitsquotienten von A260/A280 = 1,84 bzw. A260/A230 = 2,23.

Ohne die erfindungsgemäße Säule hätte man das Lysat in ca. 30 Einzelschritten zu je 700 µL auf eine handelsübliche Minispin-Säule laden müssen.

### BEISPIEL 2:

Protokoll zur Reinigung von PCR-Fragmenten
1. Aus einem 1% TAE Agarose Gel (30 min, 90V) wurde eine 5 g schwere Bande mit 20 µg Plasmid-DNA ausgeschnitten und mit 10 mL NTI Puffer (NucleoSpin Gel and PCR Clean-up, REF 740609, MACHEREY-NAGEL, Düren, Deutschland - handelsüblicher Bindepuffer mit Guanidinium thiocyanat) bei 55°C bis zur vollständigen Auflösung inkubiert.
2. Die Probe wurde über Vakuum auf die erfindungsgemäße Säule (2 Lagen Silicamembran, Polyethylenfritte als Träger) geladen und mit 5 mL Waschpuffer NT3 unter Vakuum gewaschen (NT3, NucleoSpin Gel and PCR Clean-up, REF 740609, MACHEREY-NAGEL, Düren, Deutschland - handelsüblicher Waschpuffer mit 80% Ethanol).
3. Dann wurde der Reservoirteil entfernt (abgebrochen), der untere Teil der Säule in ein Collection Tube (2 mL) überführt.
4. Die Säule wurde in einer Tischzentrifuge für 2 min bei 11.000 x g getrocknet.
5. Die Elution der DNA erfolgte in 100 µL Elutionspuffer NE (5 mM Tris/HCl) durch Zentrifugation für 1 min bei 11.000 x g.

### Ergebnis:

Die photometrische Vermessung des Eluates ergab eine Ausbeute von 12,8 µg (64%) und Reinheitsquotienten von A260/A280 = 1,85 bzw. A260/A230 = 2,06.

Ohne die erfindungsgemäße Säule hätte man das Lysat in ca. 20 Einzelschritten zu je 700 µL auf eine handelsübliche Minispin Säule laden müssen.

### BEISPIEL 3:

Protokoll zur Reinigung genomischer DNA aus komplexen biologischen Proben
1. 1 g Kochschinken wurde mit 2750 µL Lysepuffer CF und 50 µL Proteinase K für 3 h bei 65°C inkubiert (Lysepuffer CF: NucleoSpin Food, REF 740945, MACHEREY-NAGEL, Düren, Deutschland - handelsüblicher Lysepuffer mit SDS).
2. Unzersetztes Probenmaterial wurde für 10 min bei 10.000 x g pelletiert. Der klare Überstand wurde mit 1 Volumen Bindepuffer C4 und einem Volumen Ethanol versetzt und gemischt (C4: NucleoSpin Food, REF 7409454, MACHEREY-NAGEL, Düren, Deutschland - handelsüblicher Bindepuffer mit Guanidinium hydrochlorid)
3. Die Probe wurde über Vakuum auf die erfindungsgemäße Säule (3 Lagen Silicamembran, Polyethylenfritte als Träger) geladen und mit 5 mL Waschpuffer CQW, sowie 2x 5 mL Waschpuffer C5 unter Vakuum gewaschen (NucleoSpin Food, REF 740945, MACHEREY-NAGEL, Düren, Deutschland - CQW: handelsüblicher Waschpuffer mit Guanidinium hydrochlorid und Ethanol; C5: handelsüblicher Alkohol-Waschpuffer mit 80 % Ethanol).
4. Dann wurde der Reservoirteil entfernt (abgebrochen), der untere Teil der Säule in ein Collection Tube (2 mL) überführt.
5. Die Säule wurde in einer Tischzentrifuge für 2 min bei 11.000 x g getrocknet.
6. Die Elution der DNA erfolgte in 2x100 µL Elutionspuffer CE (5 mM Tris/HCl) durch Zentrifugation für 1 min bei 11.000 x g.

### Ergebnis:

Die photometrische Vermessung des Eluates ergab eine Ausbeute von 120 µg und Reinheitsquotienten von A260/A280 = 1,91 bzw. A260/A230 = 2,19.

Ohne die erfindungsgemäße Säule hätte man das Lysat in ca. 15 Einzelschritten zu je 700 µL auf eine handelsübliche Minispin-Säule laden müssen.

### BEISPIEL 4:

Protokoll zur Reinigung genomischer DNA humanem Plasma
1. 2,4 mL Plasma wurde mit 3,6 mL Puffer BB versetzt (Bindepuffer BB: NucleoSpin Plasma XS, REF 740900, MACHEREY-NAGEL, Düren, Deutschland - handelsüblicher Bindepuffer mit Guanidinium Thiocyanat und Ethanol).
2. Die Probe wurde über Vakuum auf die erfindungsgemäße Säule geladen (3 Lagen Silicamembran, Polyethylenfritte als Träger). Die folgenden Waschschritte erfolgten in einer Tischzentrifuge.
3. Der Reservoirteil der Säule wurde entfernt (abgebrochen), der untere Teil der Säule in ein 2 mL Collection Tube eingesteckt, mit 500 µL Waschpuffer WB beladen (handelsüblicher Waschpuffer, Tris/HCl, >60% Ethanol) und für 30 s bei 11.000xg zentrifugiert.
4. Das Collection Tube mit dem Durchlauf wurde verworfen und ein zweites Mal mit 250 µL Waschpuffer WB gewaschen.
5. Anschließend wurde die Säule durch Zentrifugation bei 11.000xg für 3 min getrocknet.
6. Die Elution der DNA erfolgte in 200 µL Elutionspuffer BE (5 mM Tris/HCl) durch Zentrifugation für 30 s bei 11,000 x g.

Parallel wurden 2 weitere Ansätze durchgeführt. Zum einen wurden gemäß dem NucleoSpin Plasma XS High Sens Protokoll (MN, REF 740900) 240 µL Plasma mit 360 µL Bindepuffer BB versetzt. Dies entspricht 1/10 der Volumina aus dem o.g. Ausführungsbeispiel. Als Bindesäule dienten konventionelle NucleoSpin Plasma XS Minispin-Säulen. Die Beladung und alle übrigen Schritte erfolgten in einer Tischzentrifuge unter identischen Bedingungen.

In einem weiteren Ansatz wurden wie im o.g. Ausführungsbeispiel 2,4 mL Plasma mit 3,6 mL Bindepuffer BB versetzt. Als Säule wurde hier jedoch eine große Trichtersäule (MACHEREY-NAGEL Funnel Column) eingesetzt. Die Prozessierung erfolgte in einer Standzentrifuge. Die einzelnen Schritte wurden wie folgt durchgeführt:
1. Waschschritt 5 mL WB (1.000xg, 3 min)
2. Waschschritt 2,5 mL WB und Trocknen (3.000xg, 3 min)
3. Elution: 200 µL BE, 3.000xg 3 min.

Die DNA-Quantifizierung erfolgte mittels quantitativer rtPCR (CyNamo Capillary SYBR Green qPCR Kit).

### Ergebnis:

Es konnte mit allen drei Formaten DNA aus Plasma isoliert werden. Die DNA-Ausbeute betrug für die NucleoSpin Plasma XS Säule 13 ng, für die große Trichtersäule (Funnel Column) 108 ng und für die erfindungsgemäße Säule 194 ng. Entgegen der Minispin-Säule konnte mit der erfindungsgemäßen Säule die 10-fache Plasmamenge aufbereitet werden (240 µL vs. 2,4 mL, DNA-Ausbeute 13 ng vs. 194 ng). Das erfindungsgemäße Verfahren ist zudem der großen Trichtersäule bei gleichem Probenvolumen (in beiden Fällen 2,4 mL) in Bezug auf DNA-Ausbeute (194 vs. 108 ng) und DNA-Konzentration (0,97 vs 0,54 ng/µL) überlegen.

### BEISPIEL 5:

Protokoll zur Reinigung von RNA
1. RNA wurde nach einem Clean-up Protokoll aufgereinigt. Die RNA lag vorgereinigt in Wasser mit einer Konzentration von 1 ng/µL vor. 3 mL der RNA-Lösung wurden mit 3 mL Puffer RCU versetzt (Bindepuffer RCU: NucleoSpin RNA Clean-up XS Kit, REF 740903, MACHEREY-NAGEL, Düren, Deutschland - handelsüblicher Bindepuffer mit Guanidinium Thiocyanat und Ethanol).
2. Die Probe wurde über Vakuum auf die erfindungsgemäße Säule (3 Lagen Silicamembran, Polyethylenfritte als Träger) geladen. Die folgenden Waschschritte erfolgten in einer Tischzentrifuge.
3. Der Reservoirteil der Säule wurde entfernt (abgebrochen), der untere Teile der Säule in ein 2 mL Collection Tube eingesteckt, mit 400 µL Waschpuffer RA3 beladen (handelsüblicher Waschpuffer, Tris/HCl, >70% Ethanol) und für 30 s bei 11.000xg zentrifugiert.
4. Das Collection Tube mit dem Durchlauf wurde verworfen und ein zweites mal mit 200 µL Waschpuffer RA3 gewaschen.
5. Die Elution der RNA erfolgte in 100 µL Wasser durch Zentrifugation für 30 s bei 11,000 x g.

Parallel wurden 300 µL der RNA-Lösung mit 300 µL RCU versetzt und mit konventionellen Minispin Säulen aus dem NucleoSpin RNA Kit (MACHEREY-NAGEL, Düren, Deutschland, REF 740955) unter Zentrifugation prozessiert. Alle übrigen Schritte erfolgten gemäß dem oben beschriebenen Protokoll.

Die Quantifizierung der RNA erfolgte mittels RiboGreen.

### Ergebnis:

Die RNA Ausbeute mit den erfindungsgemäßen Säulen betrug 5,5 µg, während die Ausbeute mit den Minispin Säulen 0,7 µg ergab. Das Scale-up im Faktor 10 in Bezug auf das Ausgangsmaterial (300 µL bei der Minispin-Säule, 3 mL bei der erfindungsgemäßen Säule) spiegelt sich damit auch ansatzweise bei der RNA Ausbeute wieder.

### BEISPIEL 6:

Protokoll zur Fällung und Reinigung von Plasmid DNA
1. 6 µg pcDNA3.1 werden in 5 mM Tris/HCl vorgelegt und mit Wasser auf 1, 2, 4, 8, 16 mL aufgefüllt.
2. Die DNA der Proben wird durch Zugabe von 1 Volumen Polyethylenglycol (20% PEG 8000, 2,5 M NaCl) und Inkubation for 2 h bei 4°C gefällt.
3. Der Reaktionsansatz wurde auf die erfindungsgemäße Säule aufgetragen und durch Anlegen von Vakuum (-300 mbar) abgesaugt.
4. Der Reservoirteil der Säule wurde entfernt (abgebrochen) und die Säule unter Vakuum durch Zugabe von 2x 700 µL Waschpuffer A4 (handelsüblicher Alkohol-Waschpuffer mit 80 % Ethanol) gewaschen.
5. Der untere Teile der Säule wurde in ein 2 mL Collection Tube eingesteckt und für 30 s bei 11.000xg zur Trocknung in einer Tischzentrifuge zentrifugiert.
6. Die Elution der DNA erfolgte in 2x 200 µL 5 mM Tris/HCl durch Zentrifugation für 30 s bei 11,000xg.

### Ergebnis:

Die DNA Ausbeute betrug für 1, 2, 4, 8, und 16 mL 5,8, 5,4, 4,8 und 4,5 µg DNA. Damit wurden Ausbeuten zwischen 99 und 72% erzielt.

## Patentansprüche

1. Vorrichtung (1) zur Aufreinigung von Nukleinsäuren aus einem einteilig ausgebildeten Hohlkörper (2) mit einem eine Einlassöffnung (5) umfassenden oberen Abschnitt (3) und einem eine Auslassöffnung (6) umfassenden unteren Abschnitt (4), wobei in dem Hohlkörper (2) zumindest eine Nukleinsäure-bindende Matrix (7) angeordnet ist,
**dadurch gekennzeichnet, dass**
zwischen dem oberen Abschnitt (3) und dem unteren Abschnitt (4) eine Sollbruchstelle (10) vorgesehen und die Nukleinsäure-bindende Matrix (7) im unteren Abschnitt (4) angeordnet ist, wobei das Volumen des oberen Abschnitts (3) zumindest dem 5-fachen des Volumens des unteren Abschnitts (4) entspricht.

2. Vorrichtung nach Anspruch 1, **dadurch gekennzeichnet, dass** das Volumen des oberen Abschnitts (3) wenigstens dem 20-fachen des Volumens des unteren Abschnitts (4) entspricht, vorzugsweise wenigstens dem 40-fachen, besonders bevorzugt dem 50-fachen, ganz besonders bevorzugt dem wenigstens 55-fachen.

3. Vorrichtung nach Anspruch 1 oder 2, **dadurch gekennzeichnet, dass** der obere Abschnitt (3) und der untere Abschnitt (4) unabhängig voneinander einen runden oder rechteckigen, insbesondere quadratischen Querschnitt besitzen.

4. Vorrichtung nach einem der vorstehenden Ansprüche, **dadurch gekennzeichnet, dass** der obere Abschnitt (3) und der untere Abschnitt (4) unabhängig voneinander eine zylindrische oder konische Form besitzen.

5. Vorrichtung nach einem der vorstehenden Ansprüche, **dadurch gekennzeichnet, dass** der Hohlkörper (2) aus einem Kunststoff hergestellt ist, wobei der Kunststoff insbesondere ausgewählt ist aus Polyolefinen wie Polyethylen oder Polypropylen, aus biobasierten Kunststoffen wie Polyhydroxybuttersäure oder Polylactaten, Polyamiden wie Nylon, Polyimiden, Acetalen, Polyvinylchlorid, Polytetrafluorethylen, Polyestern, Polycarbonaten, Polymethyl(meth)acrylaten, Acrylnitril-Butatien-Styrol Terpolymerisat (ABS), Polystyrol oder beliebigen Mischungen und/ oder Copolymeren von diesen.

6. Vorrichtung nach einem der vorstehenden Ansprüche, **dadurch gekennzeichnet, dass** der Hohlkörper (2) über ein Spritzgussverfahren, durch Blasformen, Gießtechnik in Silikonformen, Methoden des Rapid-Prototyping wie 3D-Druck, Fused Deposite Modeling, Lasersintern oder Elektronenstrahlschmelzen, oder durch zerspanende, formgebende Bearbeitungsverfahren, wie Drehen, Fräsen, Bohren, Sägen und Schleifen hergestellt ist.

7. Vorrichtung nach einem der vorstehenden Ansprüche, **dadurch gekennzeichnet, dass** die Nukleinsäure-bindende Matrix (7) eine Membran, ein Faserfilter, eine Fritte, und/ oder ein partikulärer Filter wie ein Anionentauscherharz ist, wobei die Membran insbesondere eine Silica-, Glas- oder Quartzfasermembran ist.

8. Vorrichtung nach Anspruch 7, **dadurch gekennzeichnet, dass** die Membran derart ausgewählt ist, dass sie ein mittleres Partikelrückhaltevermögen von 0,1 bis 5 µm gemessen nach DIN EN 1822-3 (Januar 2011) aufweist.

9. Vorrichtung nach einem der vorstehenden Ansprüche, **dadurch gekennzeichnet, dass** die Nukleinsäure-bindende Matrix (7) auf einem Träger (8) fixiert ist, insbesondere auf einer Trägerfritte, wobei die Fixierung vorzugsweise über einen Spannring (9) erfolgt.

10. Vorrichtung nach einem der vorstehenden Ansprüche, **dadurch gekennzeichnet, dass** die Sollbruchstelle (10) durch eine im Wesentlichen durchgehend den Hohlkörper (2) umlaufende Schwächungslinie ausgebildet ist, insbesondere als die Wandungsstärke des Hohlkörpers (2) reduzierende Nut, vorzugsweise in Form einer Keilnut.

11. Vorrichtung nach einem der vorstehenden Ansprüche, **dadurch gekennzeichnet, dass** die Sollbruchstelle (10) derart ausgestaltet ist, dass sie eine Trennung des oberen Abschnitts (3) vom unteren Abschnitt (4) ohne Zuhilfenahme von Werkzeugen ermöglicht.

12. Vorrichtung nach einem der vorstehenden Ansprüche, **dadurch gekennzeichnet, dass** die Auslassöffnung (6) derart ausgestaltet ist, dass sie sich zum Aufstecken auf eine Vakuumkammer eignet, wobei die Auslassöffnung (6) vorzugsweise als Luer-Male Adapter ausgestaltet ist.

13. Verfahren zur Herstellung einer Vorrichtung zur Aufreinigung von Nukleinsäuren nach einem der Ansprüche 1 bis 12, wobei ein einteiliger Hohlkörper (2) mit einer Einlassöffnung (5) und einer Auslassöffnung 6) und einer Sollbruchstelle (10) erzeugt und anschließend in den Hohlkörper (2) zwischen der Sollbruchstelle (10) und der Auslassöffnung (6) zumindest eine Nukleinsäure-bindende Matrix (7) angeordnet wird.

14. Verfahren zur Aufreinigung von Nukleinsäuren aus einer flüssigen Nukleinsäure-haltigen Probe mithilfe einer Vorrichtung nach einem der Ansprüche 1 bis 12 umfassend die folgenden Schritte:
a) Bereitstellen der flüssigen Nukleinsäuren enthaltenden Probe und Einstellung der Bindebedingungen, damit die Nukleinsäuren an die Nukleinsäure-bindende Matrix (7) binden können;
b) Überführen der Probe in die Vorrichtung (1) durch die Einlassöffnung (5) der Vorrichtung (1);
c) Hindurchführen der Probe durch die Nukleinsäure-bindende Matrix (7), wobei die Nukleinsäuren an die Nukleinsäure-bindende Matrix (7) binden und wobei das Hindurchführen insbesondere unter Anlegen von Vakuum an der Auslassöffnung (6) der Vorrichtung (1) erfolgt;
d) optionales Waschen der Nukleinsäure-bindenden Matrix (7);
e) Trennen des oberen Abschnitts (3) vom unteren Abschnitt (4) entlang der Sollbruchstelle (10), insbesondere durch händisches Abbrechen;
f) Optionales Waschen der Nukleinsäure-bindenden Matrix (7);
g) Elution der Nukleinsäuren von der Nukleinsäure-bindenden Matrix (7) und Auffangen der eluierten Nukleinsäuren in einem separaten Auffanggefäß, wobei die Elution insbesondere in einer Zentrifuge durchgeführt wird.

15. Kit zur Aufreinigung von Nukleinsäuren aus einer Nukleinsäure-haltigen flüssigen Probe umfassend eine Vorrichtung (1) nach einem der Ansprüche 1 bis 12, sowie eine Bedienungsanleitung zur Durchführung eines Verfahrens nach Anspruch 14, und/ oder zur Aufreinigung von Nukleinsäuren geeignete Mittel, wie mindestens einen Lyse- und/oder Bindepuffer, Waschpuffer und/oder Elutionspuffer.

## Claims

1. Device (1) for purifying nucleic acids composed of a one-piece hollow body (2) comprising an upper portion (3) having an inlet port (5) and a lower portion (4) having an outlet port (6), wherein at least one nucleic acid-binding matrix (7) is arranged within the hollow body (2),
**characterized in that**
between the upper portion (3) and the lower portion (4) a predetermined breaking point (10) is provided and the nucleic acid-binding matrix (7) is arranged in the lower portion (4), wherein the volume of upper portion (3) corresponds to a volume that is at least 5-fold the volume of lower portion (4).

2. Device according to Claim 1, **characterized in that** the volume of upper portion (3) corresponds to a volume that is at least 20-fold the volume of lower portion (4), preferably at least 40-fold, more preferably at least 50-fold, and most preferably at least 55-fold.

3. Device according to Claim 1 or 2, **characterized in that** the upper portion (3) and lower portion (4) independently of one another have a round or rectangular, in particular square cross section.

4. Device according to one of the preceding Claims, **characterized in that** the upper portion (3) and lower portion (4) independently of one another have a cylindrical or conical form.

5. Device according to one of the preceding Claims, **characterized in that** the hollow body (2) is manufactured of a plastic, wherein the plastic is in particular selected from polyolefins such as polyethylene or polypropylene, from bio-based plastics such as polyhydroxybutyric acid, or polylactates, polyamides such as nylon, polyimides, acetals, polyvinyl chloride, polytetrafluoroethylene, polyesters, polycarbonates, polymethyl(meth)acrylates, acrylonitrile butatien styrene terpolymer (ABS), polystyrene or any desired mixtures and/or copolymers thereof.

6. Device according to one of the preceding Claims, **characterized in that** the hollow body (2) is manufactured by means of an injection molding process, blow molding, casting technique with silicone forms, methods of rapid prototyping such as 3D printing, fused deposition modeling, laser sintering or electron beam melting or by means of forming machining methods such as turning, milling, drilling, sawing, and grinding.

7. Device according to one of the preceding Claims, **characterized in that** the nucleic acid-binding matrix (7) is a membrane, a fiber filter, frit and/or a particulate filter such as an anion exchange resin, wherein the membrane is comprised of a in particular silica-, glass- or quartz fiber membrane.

8. Device according to Claim 7, **characterized in that** the membrane is selected so as to comprise a mean retention of 0.1 to 5 µm measured according to DIN EN 1822-3 (January 2011).

9. Device according to one of the preceding Claims, **characterized in that** the nucleic acid-binding matrix (7) is immobilized on a carrier (8), in particular on a frit carrier, wherein the immobilization is preferably achieved by means of a clamping ring (9).

10. Device according to one of the preceding claims, wherein the predetermined breaking point (10) is formed by a weakening line that substantially encircles the hollow body (2) in a continuous form, in particular as a groove reducing the wall thickness of the hollow body (2), preferably in form of a keyway.

11. Device according to one of the preceding Claims, **characterized in that** the predetermined breaking point (10) is designed such that it enables a separation of the upper portion (3) from the lower portion (4) without the aid of tools.

12. Device according to one of the preceding Claims, **characterized in that** the outlet opening (6) is designed such that it is suitable for attachment to a vacuum chamber, wherein the outlet opening (6) is preferably configured as a luer male adapter.

13. Method for producing a device for purifying nucleic acids according to one of the Claims 1 to 12, **characterized in that** a one-piece hollow body (2) having an inlet opening (5) and an outlet opening (6) and a predetermined breaking point (10) is produced and at least one nucleic acid-binding matrix (7) is subsequently arranged within hollow body (2) between the predetermined breaking point (10) and the outlet opening (6).

14. Method for purifying nucleic acids from a liquid nucleic acid-containing sample by means of a device according to one of Claims 1 to 12 comprising the following steps:
a) Providing the sample containing the liquid nucleic acids and adjusting the binding conditions so as to achieve binding of the nucleic acids to the nucleic acid-binding matrix;
b) Transferring the sample into the device (1) through the inlet opening (5) of the device (1);
c) Passing the sample through the nucleic acid-binding matrix (7), wherein the nucleic acids bind to the nucleic acid-binding matrix (7) and wherein the passage is effected in particular by applying a vacuum to the outlet opening (6) of the device (1);
d) Optionally washing the nucleic acid-binding matrix (7);
e) Disconnecting the upper portion (3) from the lower portion (4) along the predetermined breaking point (10), in particular by manual breakage;
f) Optionally washing the nucleic acid-binding matrix (7);
g) Eluting the nucleic acids from the nucleic acid-binding matrix (7) and collecting the eluted nucleic acids in a separate collecting vessel, wherein the elution is carried out in particular in a centrifuge.

15. Kit for purifying nucleic acids from a liquid nucleic acid-containing sample comprising a device (1) according to one of Claims 1 to 12, and an operating manual for performing the method according to Claim 14, and/or agents suitable for purifying nucleic acids, such as at least one lysis- and/or binding buffer, wash buffer, and/or elution buffer.

## Revendications

1. Dispositif (1) pour la purification des acides nucléiques constitué d'un corps creux intégralement formé (2) comprenant une partie supérieure (3) ayant un orifice d'entrée (5) et une partie basse (4) ayant un orifice de sortie (6), dans lequel au moins une matrice de fixation des acides nucléiques (7) est disposée à l'intérieur du corps creux (2),
**caractérisé en ce que**
entre la partie supérieure (3) et la partie inférieure (4) un point de rupture prédéterminé (10) est fourni et la matrice de fixation des acides nucléiques (7) est disposée dans la partie inférieure (4) dans lequel le volume de la partie supérieure (3) correspond à au moins 5 fois le volume de la partie inférieure (4).

2. Dispositif selon la revendication 1, **caractérisé en ce que** le volume de la partie supérieure (3) correspond à au moins 20 fois le volume de la partie inférieure (4), préférablement au moins 40 fois, plus préférablement au moins 50 fois, et encore plus préférablement au moins 55 fois.

3. Dispositif selon la revendication 1 ou 2, **caractérisé en ce que** la partie supérieure (3) et la partie inférieure (4), indépendamment l'une de l'autre, ont une section transversale ronde ou rectangulaire, en particulier une section transversale carrée.

4. Dispositif selon l'une des revendications qui précèdent, **caractérisé en ce que** la partie supérieure (3) et la partie inférieure (4), indépendamment l'une de l'autre, ont une forme cylindrique ou conique.

5. Dispositif selon l'une des revendications qui précèdent, **caractérisé en ce que** le corps creux (2) est fabriqué en plastique, dans lequel le plastique est sélectionné en particulier à partir de polyoléfines tels que le polyéthylène ou le polypropylène, de matières plastiques d'origine biologique telles que l'acide polyhydroxybutyrique, ou des polylactates, des polyamides tels que nylon, polyimides, acétals, polychlorure de vinyle, polytétrafluoroéthylène, polyesters, polycarbonates, polyméthyl(meth)acrylates, terpolymères acrylonitrile-butadiène-styrène (ABS), polystyrène ou tous mélanges désirés et/ou copolymères de ces derniers.

6. Dispositif selon l'une des revendications qui précèdent, **caractérisé en ce que** le corps creux (2) est fabriqué au moyen d'un procédé de moulage par injection, moulage par soufflage, une technique de moulage avec formes en silicone, des méthodes de prototypage rapide telles que l'impression en trois dimensions, dépôt de filament en fusion, frittage au laser ou fusion par faisceau d'électrons, ou au moyen de méthodes d'usinages, telles que tournage, fraisage, perçage, sciage, et meulage.

7. Dispositif selon l'une des revendications qui précèdent, **caractérisé en ce que** la matrice de fixation des acides nucléiques (7) est une membrane, un filtre en fibres, un filtre fritté et/ou à particules telle qu'une résine d'échange anionique, dans laquelle la membrane est constituée d'une membrane particulière en silice, verre, ou fibres de quartz.

8. Dispositif selon la revendication 7, **caractérisé en ce que** la membrane est sélectionnée de façon à comporter une rétention moyenne de 0,1 à 5 µm mesurée selon la norme DIN EN 1822-3 (janvier 2011).

9. Dispositif selon l'une des revendications qui précèdent, **caractérisé en ce que** la matrice de fixation des acides nucléiques (7) est immobilisée sur un support (8), notamment sur un support fritté, dans lequel l'immobilisation est préférablement obtenue au moyen de bagues de serrage (9).

10. Dispositif selon l'une des revendications qui précèdent, **caractérisé en ce qu'**un point de rupture prédéterminé (10) est formé par une ligne de faiblesse qui entoure de façon substantielle le corps creux (2) dans une forme continue, notamment sous la forme d'une gorge réduisant l'épaisseur de la paroi du corps creux (2), de préférence en formant une rainure.

11. Dispositif selon l'une des revendications qui précèdent, **caractérisé en ce que** le point de rupture prédéterminé (10) est conçu de telle manière qu'il permet une séparation de la partie supérieure (3) et de la partie inférieure (4) sans l'aide d'outils.

12. Dispositif selon l'une des revendications qui précèdent, **caractérisé en ce que** l'orifice de sortie (6) est conçu de façon à ce qu'il soit possible d'y fixer une chambre à vide, dans lequel l'orifice de sortie (6) est préalablement configuré comme un adaptateur Luer mâle.

13. Méthode pour la production d'un dispositif destiné à la purification des acides nucléiques selon l'une des revendications 1 à 12, **caractérisé en ce qu'**un corps creux d'une seule pièce (2) comportant un orifice d'entrée (5) et un orifice de sortie (6) et un point de rupture prédéterminé (10) sont produits et au moins une matrice de fixation des acides nucléiques (7) est ensuite disposée dans le corps creux (2) entre le point de rupture prédéterminé (10) et l'orifice de sortie (6).

14. Une méthode pour la purification des acides nucléiques à partir d'un échantillon liquide contenant de l'acide nucléique au moyen d'un dispositif selon l'une des revendications 1 à 12 comprenant l'une des étapes suivantes :
a) Sous réserve que des acides nucléiques liquides soient contenus dans le liquide et de l'ajustement des conditions de fixation de façon à obtenir une fixation des acides nucléiques à la matrice de fixation des acides nucléiques ;
b) Transfert de l'échantillon dans le dispositif (1) via l'orifice d'entrée (5) dans le dispositif (1) ;
c) Passage de l'échantillon à travers la matrice de fixation des acides nucléiques (7), dans lequel les acides nucléiques se fixent à la matrice de fixation des acides nucléiques (7) et dans lequel le passage s'effectue en particulier en appliquant un vide à l'orifice de sortie (6) du dispositif (1) ;
d) Lavage optionnel de la matrice de fixation des acides nucléiques (7) ;
e) Déconnexion entre la partie supérieure (3) et la partie inférieure (4) le long du point de rupture prédéterminé (10), notamment en les brisant manuellement ;
f) Lavage optionnel de la matrice de fixation des acides nucléiques (7) ;
g) Élution des acides nucléiques de la matrice de fixation des acides nucléiques (7) et recueil des acides nucléiques élués dans un récipient collecteur séparé, dans lequel l'élution est assurée en particulier dans une centrifugeuse.

15. Kit pour la purification des acides nucléiques provenant d'un échantillon liquide contenant des acides nucléiques comportant un dispositif (1) selon l'une des revendications 1 à 12 et un manuel d'utilisation pour la réalisation de la méthode selon la revendication 14, et/ou des produits convenables pour la purification des acides nucléiques, tels qu'au moins un tampon de lyse et/ou de liaison, un tampon de lavage, et/ou un tampon d'élution.
